# EUROPEAN PATENT SPECIFICATION

(11) **EP 4 294 286 B1**
(45) Date of publication and mention of the grant of the patent: **19.11.2025**
(21) Application number: 22723303.8
(22) Date of filing: 25.04.2022
(51) Int. Cl.: A61B 17/12

(54) **LEFT ATRIAL APPENDAGE IMPLANT**
IMPLANTAT FÜR LINKES HERZOHR
IMPLANT D'APPENDICE AURICULAIRE GAUCHE

(30) Priority: 27.04.2021 US 202163180210 P
(43) Date of publication of application: 27.12.2023
(73) Proprietor: Boston Scientific Scimed, Inc., Maple Grove, MN 55311 (US)
(72) Inventor: NATESAN, Harishankar, Shoreview, Minnesota 55126 (US); CHEN, Jan-hung, St. Paul, Minnesota 55105 (US)
(74) Representative: Vossius & Partner Patentanwälte Rechtsanwälte mbB
(86) International application number: PCT/US2022/026174
(87) International publication number: WO 2022/232043

(56) References cited:
- WO-A1-2013/159065
- US-A1- 2014 018 841
- US-A1- 2017 156 840

## Description

### Cross-Reference to Related Applications

This application claims the benefit of priority of U.S. Provisional Application No. 63/180,210 filed April 27, 2021.

### Technical Field

The disclosure relates generally to medical devices and more particularly to medical devices that are adapted for use in percutaneous medical procedures including implantation into the left atrial appendage (LAA) of a heart.

### Background

The left atrial appendage is a small organ attached to the left atrium of the heart. During normal heart function, as the left atrium constricts and forces blood into the left ventricle, the left atrial appendage constricts and forces blood into the left atrium. The ability of the left atrial appendage to contract assists with improved filling of the left ventricle, thereby playing a role in maintaining cardiac output. However, in patients suffering from atrial fibrillation, the left atrial appendage may not properly contract or empty, causing stagnant blood to pool within its interior, which can lead to the undesirable formation of thrombi within the left atrial appendage.

Thrombi forming in the left atrial appendage may break loose from this area and enter the blood stream. Thrombi that migrate through the blood vessels may eventually plug a smaller vessel downstream and thereby contribute to stroke or heart attack. Clinical studies have shown that the majority of blood clots in patients with atrial fibrillation originate in the left atrial appendage. As a treatment, medical devices have been developed which are deployed to close off the left atrial appendage. Document WO2013159065 Al discloses an occlusion device and method for occluding an undesirable vascular structure, such as a septal defect or left atrial appendage. The occlusion device includes a lattice structure that expands from a contracted catheter-deliverable state to an expanded state that occludes the vascular structure. The lattice structure has one or more braided layers, with structural braided layers that provide structural support to the device, and occlusive layers that provide a lattice braiding or pore sizes that promote further occlusion by a biological process, such as tissue ingrowth that further occludes the affected vascular structure. Of the known medical devices and methods, each has certain advantages and disadvantages. There is an ongoing need to provide alternative medical devices and introducers as well as alternative methods for manufacturing and using medical devices and introducers.

### Summary

The presently claimed invention provides an implant for occluding a left atrial appendage according to claim 1. Further developments of the herein claimed invention are described in the dependent claims.

In one example, an implant for occluding a left atrial appendage may comprise an expandable framework including a first framework portion and a second framework portion, wherein the expandable framework is configured to shift along a longitudinal axis between a collapsed configuration and an expanded configuration. The second framework portion may be disposed radially inward of the first framework portion in the collapsed configuration. The expandable framework includes a proximal hub and a distal hub. The first framework portion may be fixedly attached to the proximal hub and the distal hub. The second framework portion may be fixedly attached to the proximal hub and the distal hub.

In addition or alternatively to any example disclosed herein, the first framework portion includes a first plurality of interconnected struts formed from a first tubular member.

In addition or alternatively to any example disclosed herein, the second framework portion includes a second plurality of interconnected struts formed from a second tubular member.

In addition or alternatively to any example disclosed herein, a portion of the second framework portion is disposed radially outward of the first framework portion in the expanded configuration.

In addition or alternatively to any example disclosed herein, the first framework portion includes at least one anchoring member extending radially outward from the first framework portion.

In addition or alternatively to any example disclosed herein, the portion of the second framework portion disposed radially outward of the first framework portion includes at least one anchoring member extending radially outward from the second framework portion.

In addition or alternatively to any example disclosed herein, the portion of the second framework portion disposed radially outward of the first framework portion is disposed adjacent a proximal shoulder of the first framework portion.

In addition or alternatively to any example disclosed herein, the portion of the second framework portion disposed radially outward of the first framework portion is disposed proximate a midsection of the first framework portion.

In addition or alternatively to any example disclosed herein, an outer surface of the second framework portion abuts an inner surface of the first framework portion in the expanded configuration.

In addition or alternatively to any example disclosed herein, the first framework portion includes at least one anchoring member extending radially outward from the first framework portion.

In addition or alternatively to any example disclosed herein, the at least one anchoring member is aligned longitudinally with the outer surface of the second framework portion abutting the inner surface of the first framework portion.

In addition or alternatively to any example disclosed herein, the outer surface of the second framework portion abuts the inner surface of the first framework portion adjacent a proximal shoulder of the first framework portion.

In addition or alternatively to any example disclosed herein, the outer surface of the second framework portion abuts the inner surface of the first framework portion proximate a midsection of the first framework portion.

In addition or alternatively to any example disclosed herein, the implant may further comprise an occlusive element disposed on the expandable framework.

In addition or alternatively to any example disclosed herein, a system for occluding a left atrial appendage may comprise a delivery sheath having a lumen; an implant for occluding the left atrial appendage, the implant comprising: an expandable framework including a first framework portion and a second framework portion, wherein the expandable framework is configured to shift along a longitudinal axis between a collapsed configuration and an expanded configuration; wherein the second framework portion is disposed radially inward of the first framework portion in the collapsed configuration; wherein the expandable framework includes a proximal hub and a distal hub; wherein the first framework portion is fixedly attached to the proximal hub and the distal hub; wherein the second framework portion is fixedly attached to the proximal hub and the distal hub; and a core wire releasably securable to the proximal hub of the implant.

In addition or alternatively to any example disclosed herein, the first framework portion includes a first plurality of interconnected struts cut from a first tubular member and the second framework portion includes a second plurality of interconnected struts cut from a second tubular member. A proximal end of the second tubular member is concentrically disposed within a proximal end of the first tubular member.

In addition or alternatively to any example disclosed herein, the second framework portion is more compliant than the first framework portion.

In addition or alternatively to any example disclosed herein, the expandable framework includes at least one anchoring member extending radially outward therefrom.

In addition or alternatively to any example disclosed herein, the expandable framework is self-biased toward the expanded configuration.

In addition or alternatively to any example disclosed herein, an implant for occluding a left atrial appendage may comprise an expandable framework including a first framework portion cut from a first tubular member and a second framework portion cut from a second tubular member, wherein the expandable framework is configured to shift along a longitudinal axis between a collapsed configuration and an expanded configuration; and an occlusive element disposed radially outward of the expandable framework. The first tubular member has a first outer diameter and the second tubular member has a second outer diameter less than the first outer diameter. The second framework portion is disposed radially inward of the first framework portion in the collapsed configuration. The expandable framework includes a proximal hub and a distal hub. A proximal end of the first framework portion is secured to the proximal hub and a distal end of the first framework portion is secured to the distal hub. A proximal end of the second framework portion is secured to the proximal hub and a distal end of the second framework portion is secured to the distal hub.

The above summary of some embodiments, aspects, and/or examples is not intended to describe each embodiment or every implementation of the present disclosure. The figures and the detailed description more particularly exemplify aspects of these embodiments.

### Brief Description of the Drawings

The disclosure may be more completely understood in consideration of the following detailed description in connection with the accompanying drawings, in which:
FIGS. 1-2 are side views of an example system for occluding a left atrial appendage;
FIG. 3 illustrates selected aspects of an implant for occluding a left atrial appendage;
FIG. 4 illustrates selected aspects of the implant of FIG. 3;
FIG. 5 illustrates selected aspects of the implant of FIG. 3;
FIG. 6 is a partial sectional view illustrating aspects of the implant of FIG. 3 in a collapsed configuration; and
FIG. 7 is a partial sectional view illustrating aspects of the implant of FIG. 3 in an expanded configuration;
FIG. 8 is a partial sectional view illustrating aspects of the implant of FIG. 3 in an expanded configuration;
FIG. 9 is a partial sectional view illustrating aspects of the implant of FIG. 3 in an expanded configuration; and
FIG. 10 is a partial sectional view illustrating aspects of the implant of FIG. 3 in an expanded configuration.

While aspects of the disclosure are amenable to various modifications and alternative forms, examples are shown in the drawings and described herein. It should be understood, however, that the intention is not to limit aspects of the disclosure to the particular embodiments described.

### Detailed Description

The following description should be read with reference to the drawings, which are not necessarily to scale, wherein like reference numerals indicate like elements throughout the several views. The detailed description and drawings are intended to illustrate but not limit the present disclosure. Those skilled in the art will recognize that the various elements described and/or shown may be arranged in various combinations and configurations without departing from the scope of the disclosure. The detailed description and drawings illustrate example embodiments of the disclosure. However, in the interest of clarity and ease of understanding, while every feature and/or element may not be shown in each drawing, the feature(s) and/or element(s) may be understood to be present regardless, unless otherwise specified.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). **In** many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the present disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device. Still other relative terms, such as "axial", "circumferential", "longitudinal", "lateral", "radial", etc. and/or variants thereof generally refer to direction and/or orientation relative to a central longitudinal axis of the disclosed structure or device.

The term "extent" may be understood to mean a greatest measurement of a stated or identified dimension, unless the extent or dimension in question is preceded by or identified as a "minimum", which may be understood to mean a smallest measurement of the stated or identified dimension. For example, "outer extent" may be understood to mean an outer dimension, "radial extent" may be understood to mean a radial dimension, "longitudinal extent" may be understood to mean a longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage, while a "minimum extent" may be considered a smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete structures or elements together.

The terms "transaortic valve implantation" and "transcatheter aortic valve implantation" may be used interchangeably and may each be referred to using the acronym "TAVI". The terms "transaortic valve replacement" and "transcatheter aortic valve replacement" may be used interchangeably and may each be referred to using the acronym "TAVR".

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The left atrial appendage may be attached to and in fluid communication with a left atrium of a patient's heart. In some patients, the left atrial appendage may have a complex geometry and/or irregular surface area. Those of skill in the art will also recognize that the medical devices and methods disclosed herein may be adapted for various sizes and shapes of the left atrial appendage, as necessary. The left atrial appendage may include a generally longitudinal axis arranged along a depth of a main body of the left atrial appendage. The main body may include a wall and an ostium forming a proximal mouth. In some embodiments, a lateral extent of the ostium and/or the wall may be smaller or less than a depth of the main body along the longitudinal axis, or a depth of the main body may be greater than a lateral extent of the ostium and/or the wall. In some embodiments, the left atrial appendage may include a tail-like element associated with a distal portion of the main body, which element may protrude radially or laterally away from the main body.

The following figures illustrate selected components and/or arrangements of an implant for occluding the left atrial appendage, a system for occluding the left atrial appendage, and/or methods of using the implant and/or the system. It should be noted that in any given figure, some features may not be shown, or may be shown schematically, for simplicity. Additional details regarding some of the components of the implant and/or the system may be illustrated in other figures in greater detail. While discussed in the context of occluding the left atrial appendage, the implant and/or the system may also be used for other interventions and/or percutaneous medical procedures within a patient. Similarly, the devices and methods described herein with respect to percutaneous deployment may be used in other types of surgical procedures, as appropriate. For example, in some examples, the devices may be used in a nonpercutaneous procedure. Devices and methods in accordance with the disclosure may also be adapted and configured for other uses within the anatomy.

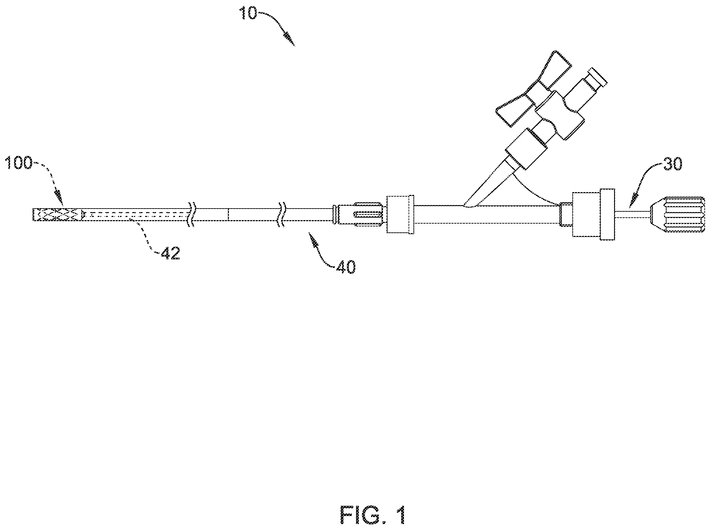

FIGS. 1-2 illustrate selected components and/or arrangements of a system 10 for occluding a left atrial appendage. It should be noted that in any given figure, some features of the system 10 may not be shown, or may be shown schematically, for simplicity. Additional details regarding some of the components of the system 10 may be illustrated in other figures in greater detail. The system 10 may be used to percutaneously deliver and/or deploy a variety of medical implants (e.g., a cardiovascular medical implant, an occlusive medical implant, a replacement heart valve implant, etc.) to one or more locations within the anatomy, including but not limited to, in some embodiments, the heart.

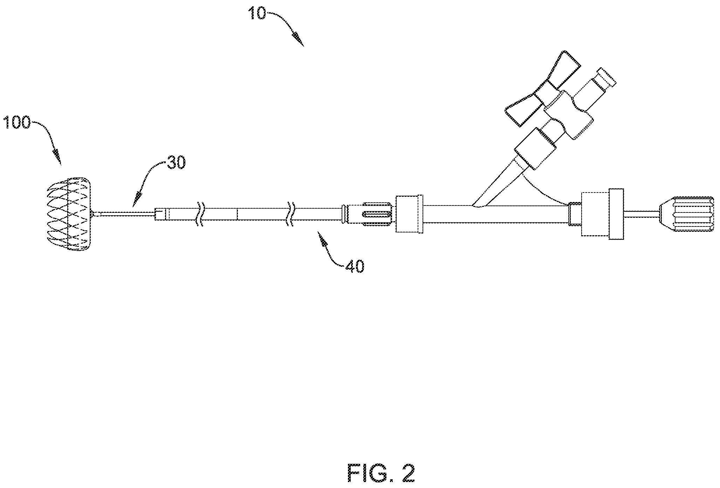

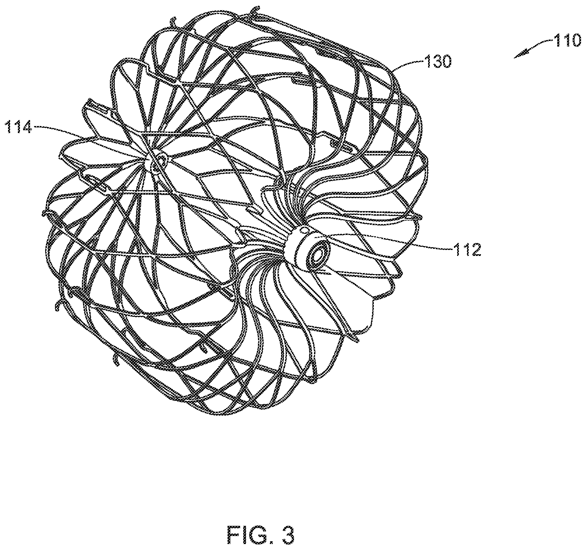

The system 10 may include a delivery sheath 40 having a lumen 42 extending from a proximal opening to a distal opening, a core wire 30 slidably disposed within the lumen 42, and an implant 100 for occluding the left atrial appendage. The implant 100 may include an expandable framework 110 (e.g., FIG. 3) configure to shift between a collapsed configuration (e.g., FIG. 1), wherein the implant 100 is disposed within the lumen 42 proximate the distal opening in the collapsed configuration, and an expanded configuration (e.g., FIG. 2), wherein the implant 100 and/or the expandable framework 110 is configured to shift between the collapsed configuration and the expanded configuration when the implant 100 is disposed distal of the distal opening of the lumen 42 and/or the delivery sheath 40, and/or when the implant 100 is unconstrained by the delivery sheath 40. In at least some embodiments, the expandable framework 110 may be self-biased toward the expanded configuration.

The implant 100 may be disposed at and/or releasably securable to a distal portion of the core wire 30. The core wire 30 may be slidably and/or rotatably disposed within the lumen 42 of the delivery sheath 40. In some embodiments, a proximal end of the core wire 30 may extend proximally of a proximal end of the delivery sheath 40 and/or the proximal opening of the lumen 42 for manual manipulation by a clinician or practitioner. In some embodiments, the implant 100 may be removably attached, joined, secured, or otherwise connected to the distal end of the core wire 30. The core wire 30 may be configured to and/or may be capable of axially translating the implant 100 relative to the delivery sheath 40. The delivery sheath 40 and/or the core wire 30 may have a selected level of axial stiffness and/or pushability characteristics while also having a selected level of flexibility to permit navigation through the patient's vasculature.

Some suitable, but non-limiting, examples of materials for the system 10, the core wire 30, the delivery sheath 40, and/or the implant 100, etc. are discussed below. It is contemplated that any and/or all example implants disclosed herein may be used in accordance with and/or be associated with the example system 10 described above.

The implant 100 may comprise an expandable framework 110 configured to shift along a longitudinal axis 102 between the collapsed configuration and the expanded configuration. In the collapsed configuration, the expandable framework 110 may be axially elongated and/or radially compressed. In the expanded configuration, the expandable framework 110 may be axially shortened and/or radially expanded. The expandable framework 110 may comprise a plurality of interconnected struts defining a plurality of cells. In some embodiments, the plurality of cells may be a plurality of closed cells. In some embodiments, the plurality of cells may be a plurality of open cells. In some embodiments, the plurality of cells may include a plurality of open cells and a plurality of closed cells in various combinations and/or arrangements.

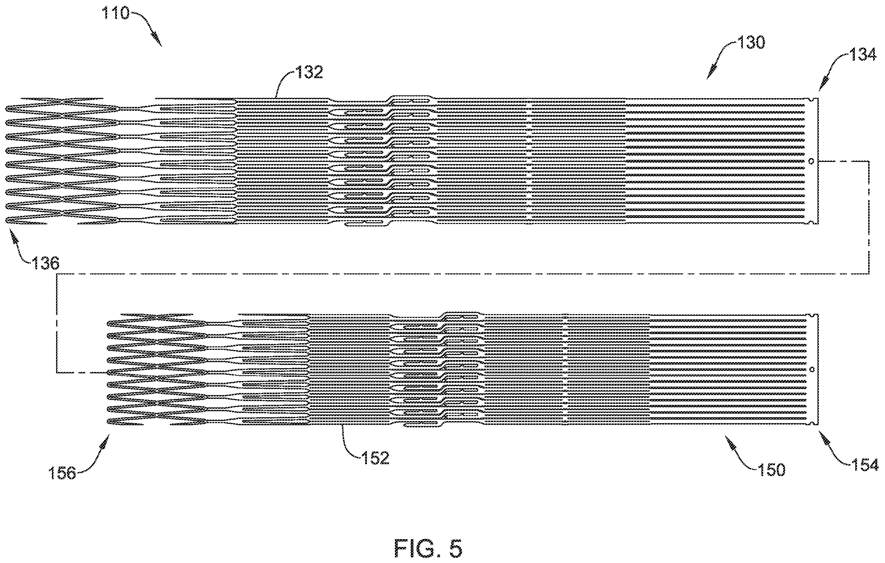

The expandable framework 110 may include a proximal hub 112 and a distal hub 114. In some embodiments, the proximal hub 112 and/or the distal hub 114 may be centered on and/or coaxial with the longitudinal axis 102. The plurality of interconnected struts may be joined together at and/or fixedly attached to the proximal hub 112 and/or the distal hub 114. The proximal hub 112 may be configured to releasably connect, secure, and/or attach the implant 100 and/or the expandable framework 110 to the core wire 30. In some embodiments, the proximal hub 112 may include internal threads configured to rotatably and/or threadably engage an externally threaded distal end of the core wire 30. Other configurations for releasably securing the implant 100 to the core wire 30 are also contemplated. The expandable framework 110 may include a first framework portion 130 (e.g., FIGS. 3, 5-6) and a second framework portion 150 (e.g., FIGS. 5-6), which elements are described in more detail herein. As noted above, some features are not shown in every figure to improve clarity.

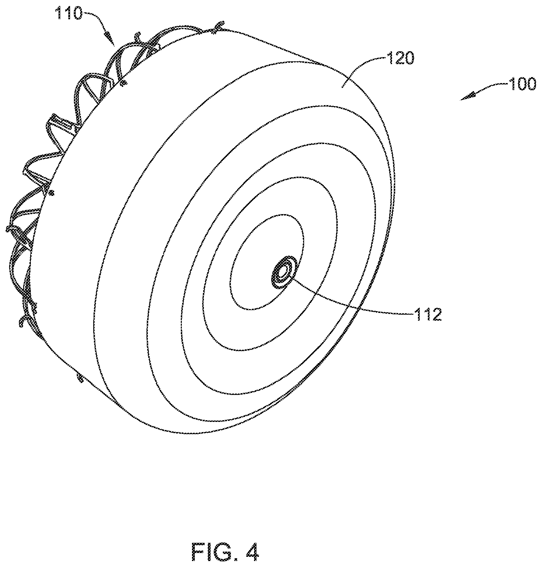

In some embodiments, the implant 100 may optionally include an occlusive element 120 connected to, disposed on, disposed over, disposed about, and/or disposed radially outward of at least a portion of the expandable framework 110 and/or the plurality of interconnected struts, as seen in FIG. 4. In some embodiments, the occlusive element 120 may be attached to the proximal hub 112 and/or may be attached to the expandable framework at the proximal hub 112. In some embodiments, the occlusive element 120 may extend radially outward from and/or may extend distally from the proximal hub 112. In some embodiments, the occlusive element 120 may be attached and/or secured to the expandable framework 110 at a plurality of discrete locations.

In some embodiments, the occlusive element 120 may include a membrane, a fabric, a mesh, a tissue element, or another suitable construction. In some embodiments, the occlusive element 120 may be porous. In some embodiments, the occlusive element 120 may be non-porous. In some embodiments, the occlusive element 120 may be permeable or impermeable to blood and/or other fluids, such as water. In some embodiments, the occlusive element 120 may be designed, sized, and/or configured to prevent thrombus and/or embolic material from passing out of the left atrial appendage into the left atrium and/or the patient's bloodstream. In some embodiments, the occlusive element 120 (e.g., the membrane, the fabric, or the tissue element, etc.) promotes endothelization after implantation, thereby effectively removing the target site (e.g., the left atrial appendage, etc.) from the patient's circulatory system. Some suitable, but non-limiting, examples of materials for the occlusive element 120 are discussed below.

FIG. 5 schematically illustrates selected aspects of the implant 100. It shall be noted that patterns, arrangements, and/or relative positioning of features shown but not explicitly discussed herein are merely exemplary and are not intended to be limiting. In some embodiments, the expandable framework 110 may include a first framework portion 130 and a second framework portion 150. The second framework portion 150 may be disposed radially inward of the first framework portion 130 in the collapsed configuration.

The first framework portion 130 may include a first plurality of interconnected struts 132 formed and/or cut from a first tubular member. The first framework portion 130 may have a proximal end 134 and a distal end 136. In some embodiments, the first framework portion 130 and/or the first plurality of interconnected struts 132 may be integrally formed and/or cut from a unitary member. In some embodiments, the first framework portion 130 and/or the first plurality of interconnected struts 132 may be integrally formed and/or cut from a unitary tubular member and subsequently formed and/or heat set to a desired shape in the expanded configuration. In some embodiments, the first framework portion 130 and/or the first plurality of interconnected struts 132 may be integrally formed and/or cut from a unitary flat member or sheet, and then rolled or formed into a tubular structure and subsequently formed and/or heat set to the desired shape in the expanded configuration. Some exemplary means and/or methods of making and/or forming the first framework portion 130 and/or the first plurality of interconnected struts 132 include laser cutting, machining, punching, stamping, electro discharge machining (EDM), chemical dissolution, etc. Other means and/or methods are also contemplated.

The second framework portion 150 may include a second plurality of interconnected struts 152 formed and/or cut from a second tubular member. The second framework portion 150 may have a proximal end 154 and a distal end 156. In some embodiments, the second framework portion 150 and/or the second plurality of interconnected struts 152 may be integrally formed and/or cut from a unitary member. In some embodiments, the second framework portion 150 and/or the second plurality of interconnected struts 152 may be integrally formed and/or cut from a unitary tubular member and subsequently formed and/or heat set to a desired shape in the expanded configuration. In some embodiments, the second framework portion 150 and/or the second plurality of interconnected struts 152 may be integrally formed and/or cut from a unitary flat member or sheet, and then rolled or formed into a tubular structure and subsequently formed and/or heat set to the desired shape in the expanded configuration. Some exemplary means and/or methods of making and/or forming the second framework portion 150 and/or the second plurality of interconnected struts 152 may include laser cutting, machining, punching, stamping, electro discharge machining (EDM), chemical dissolution, etc. Other means and/or methods are also contemplated.

As shown in FIG. 5, the second framework portion 150 may be disposed radially inward of the first framework portion 130 in the collapsed configuration. The first tubular member may have a first outer diameter and the second tubular member may have a second outer diameter less than the first outer diameter. In at least some embodiments, the second framework portion 150 may be formed and/or cut independently of the first framework portion 130 and then the second framework portion 150 may be inserted and/or positioned axially within the first framework portion 130.

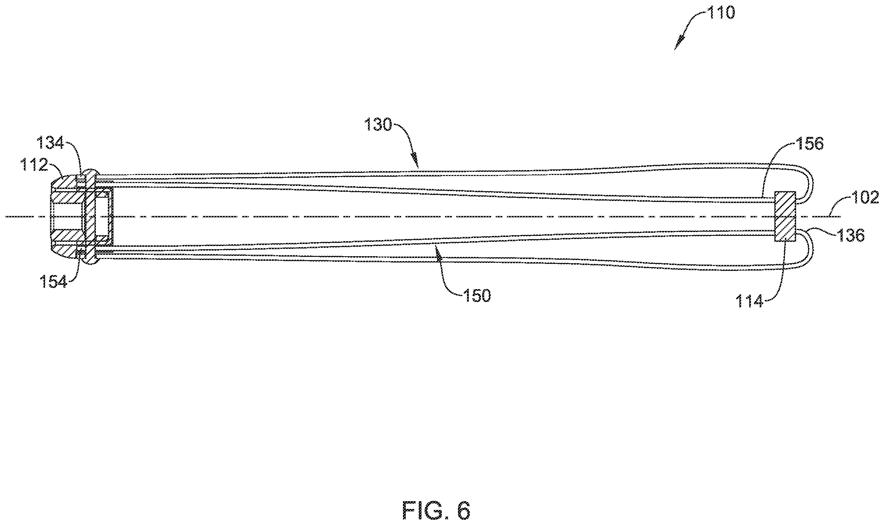

The proximal end 134 of the first framework portion 130 may be secured and/or fixedly attached to the proximal hub 112 and the distal end 136 of the first framework portion 130 may be secured and/or fixedly attached to the distal hub 114, as seen in FIG. 6. The proximal end 154 of the second framework portion 150 may be secured and/or fixedly attached to the proximal hub 112 and the distal end 156 of the second framework portion 150 may be secured and/or fixedly attached to the distal hub 114. In at least some embodiments, the proximal end 154 of the second framework portion 150 and/or the second tubular member may be concentrically disposed within the proximal end 134 of the first framework portion 130 and/or the first tubular member.

Alternatively, in some embodiments, proximal end 134 of the first framework portion 130 may be secured and/or fixedly attached to the proximal hub 112 and the distal end 136 of the first framework portion 130 may be secured and/or fixedly attached to the distal hub 114. The proximal end 154 of the second framework portion 150 may be secured and/or fixedly attached to the proximal hub 112 and the distal end 156 of the second framework portion 150 may be secured and/or fixedly attached to a second distal hub (not shown). The second distal hub may be spaced axially apart from the distal hub 114. The second distal hub may be disposed within an interior of the first framework portion 130. For example, the distal hub 114 may be a first distance axially and/or longitudinally from the proximal hub 112, and the second distal hub may be a second distance axially and/or longitudinally from the proximal hub 112, wherein the second distance is less than the first distance. In some embodiments, the second framework portion 150 may be shorter than the first framework portion 130 in the collapsed configuration and/or in the expanded configuration. In some embodiments, a connecting element may extend between the distal hub 114 and the second distal hub. In some embodiments, the connecting element may be a strut, a tether, or other element. In some embodiments, the connecting element may be a coil spring configured to bias the second distal hub toward or away from the distal hub 114. In some embodiments, as the expandable framework 110 is shift to the expanded configuration, the coil spring may urge the second distal hub away from the distal hub 114 and/or toward the proximal hub 112 to aid in shifting the second framework portion 150 to the expanded configuration. Other configurations are also contemplated.

In some embodiments, the expandable framework 110 may be compliant and substantially conform to and/or be in sealing engagement with the shape and/or geometry of a lateral wall of a left atrial appendage in the expanded configuration. In some embodiments, the implant 100 may expand to a size, extent, or shape less than or different from a maximum unconstrained extent, as determined by the surrounding tissue and/or lateral wall of the left atrial appendage. In some embodiments, reducing a thickness of various elements of the expandable framework 110 may increase the flexibility and compliance of the expandable framework 110 and/or the implant 100, thereby permitting the expandable framework 110 and/or the implant 100 to conform to the tissue around it, rather than forcing the tissue to conform to the expandable framework 110 and/or the implant 100.

In some embodiments, the first framework portion 130 may be stiffer and/or less flexible than the second framework portion 150. In some embodiments, the second framework portion 150 may be more compliant than the first framework portion 130. In some embodiments, the first framework portion 130 may be configured to shape and/or stretch the tissue of the left atrial appendage such that the lateral wall of the left atrial appendage substantially conforms to an outer shape of the first framework portion 130. As would be understood by the skilled person, anatomical features may vary in size and/or shape. In some embodiments, the left atrial appendage may have an irregular (e.g., elongated and/or oblong) cross-sectional shape. In such embodiments, the left atrial appendage may not completely conform to the outer shape of the first framework portion 130. For example, a majority of the lateral wall of the left atrial appendage may conform to the outer shape of the first framework portion 130, but a portion of the lateral wall of the left atrial appendage may be and/or remain spaced apart from the first framework portion 130 due to the irregular cross-sectional shape of the left atrial appendage, thus resulting in an incomplete and/or leaky seal between the implant 100 and the left atrial appendage.

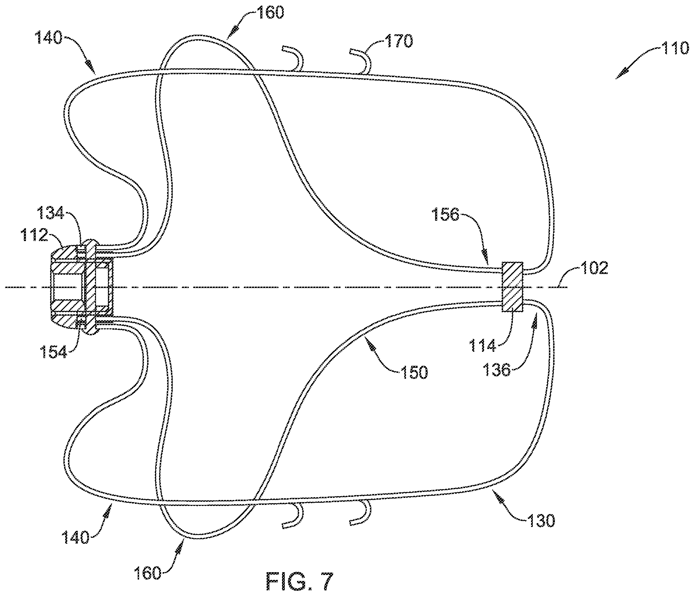

In some embodiments, the second framework portion 150 may be configured and/or heat set such that a portion 160 of the second framework portion 150 is disposed radially outward of the first framework portion 130 in the expanded configuration, as shown in FIG. 7. The second framework portion 150 may be configured to fill any gap(s) between the first framework portion 130 and the lateral wall of the left atrial appendage, thereby improving the seal between the implant 100 and the left atrial appendage. In some embodiments, the expandable framework 110 may include at least one anchoring member 170 extending radially outward therefrom. In some embodiments, the first framework portion 130 may include at least one anchoring member 170 extending radially outward from the first framework portion 130. In some embodiments, the first framework portion 130 may include at least one anchoring member 170 extending radially outward from the first framework portion 130 proximate the proximal shoulder 140. In some embodiments, the first framework portion 130 may include at least one anchoring member 170 extending radially outward from the first framework portion 130 proximate a midsection of the first framework portion 130. In some embodiments, the at least one anchoring member 170 may be configured to engage with the lateral wall of the main body of the left atrial appendage. In some embodiments, the at least one anchoring member 170 may be formed as J-shaped hooks having a free end extending in and/or directed toward a proximal direction with respect to the longitudinal axis 102 of the expandable framework 110. Other configurations are also contemplated. In some embodiments, one of, some of, and/or all of the at least one anchoring member 170 may extend through the occlusive element 120, where present, as shown in FIG. 4. In some embodiments, the portion 160 of the second framework portion 150 disposed radially outward of the first framework portion 130 may be disposed adjacent a proximal shoulder 140 of the first framework portion 130, as seen in FIG. 7.

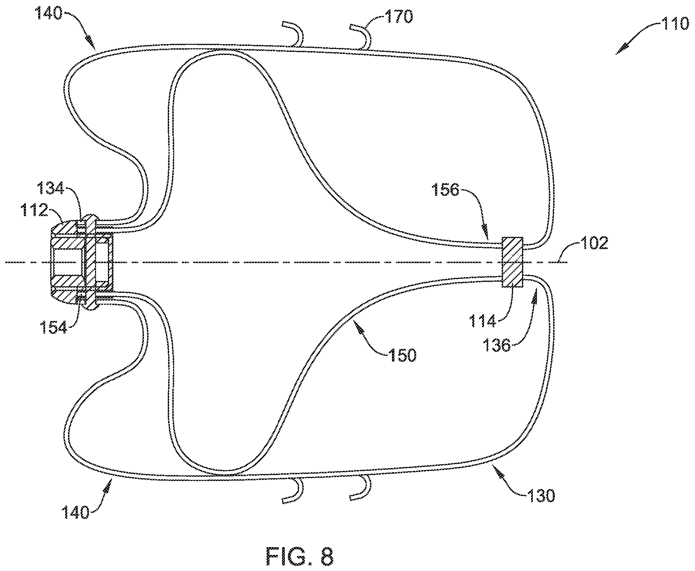

In an alternative configuration, in some embodiments, an outer surface of the second framework portion 150 may abut an inner surface of the first framework portion 130 in the expanded configuration, as seen in FIG. 8. In some embodiments, the outer surface of the second framework portion 150 may abut the inner surface of the first framework portion 130 adjacent the proximal shoulder 140 of the first framework portion 130 in the expanded configuration. In some embodiments, the second framework portion 150 may be configured to support the first framework portion 130 at and/or adjacent to the proximal shoulder 140. In some embodiments, the second framework portion 150 may be configured to increase a radially outward force exerted on the lateral wall of the left atrial appendage by the first framework portion 130 at and/or adjacent to the proximal shoulder 140 to improve shaping and/or conformation of the lateral wall of the left atrial appendage to the outer shape of the first framework portion 130. In some embodiments, the first framework portion 130 may include at least one anchoring member 170 extending radially outward from the first framework portion 130 proximate the proximal shoulder 140. In some embodiments, the first framework portion 130 may include at least one anchoring member 170 extending radially outward from the first framework portion 130 proximate a midsection of the first framework portion 130. In some embodiments, the at least one anchoring member 170 may be configured to engage with the lateral wall of the main body of the left atrial appendage. In some embodiments, the at least one anchoring member 170 may be formed as J-shaped hooks having a free end extending in and/or directed toward a proximal direction with respect to the longitudinal axis 102 of the expandable framework 110. Other configurations are also contemplated. In some embodiments, one of, some of, and/or all of the at least one anchoring member 170 may extend through the occlusive element 120, where present, as shown in FIG. 4. Acording to the invention, the at least one anchoring member 170 is aligned longitudinally with the outer surface of the second framework portion 150 abutting the inner surface of the first framework portion 130. In some embodiments, the at least one anchoring member 170 may be longitudinally offset from the outer surface of the second framework portion 150 abutting the inner surface of the first framework portion 130.

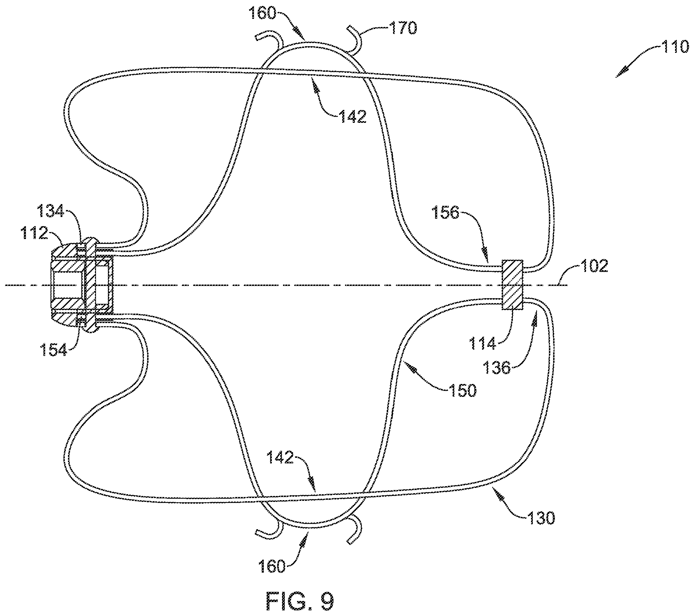

In another alternative configuration, in some embodiments, the second framework portion 150 may be configured and/or heat set such that a portion 160 of the second framework portion 150 is disposed radially outward of the first framework portion 130 in the expanded configuration, as shown in FIG. 9. The second framework portion 150 may be configured to fill any gap(s) between the first framework portion 130 and the lateral wall of the left atrial appendage, thereby improving the seal between the implant 100 and the left atrial appendage. In some embodiments, the expandable framework 110 may include at least one anchoring member 170 extending radially outward therefrom. In some embodiments, the second framework portion 150 may include at least one anchoring member 170 extending radially outward from the second framework portion 150. In some embodiments, the second framework portion 150 may include at least one anchoring member 170 extending radially outward from the second framework portion 150 proximate the proximal shoulder 140. In some embodiments, the second framework portion 150 may include at least one anchoring member 170 extending radially outward from the second framework portion 150 proximate a midsection 142 of the first framework portion 130. In some embodiments, the at least one anchoring member 170 may be configured to engage with the lateral wall of the main body of the left atrial appendage. In some embodiments, the at least one anchoring member 170 may be formed as J-shaped hooks having a free end extending in and/or directed toward a proximal direction with respect to the longitudinal axis 102 of the expandable framework 110. Other configurations are also contemplated. In some embodiments, one of, some of, and/or all of the at least one anchoring member 170 may extend through the occlusive element 120, where present, as shown in FIG. 4. In some embodiments, the portion 160 of the second framework portion 150 disposed radially outward of the first framework portion 130 may be disposed proximate the midsection 142 of the first framework portion 130, as seen in FIG. 9. In some embodiments, the second framework portion 150 may be configured to improve fixation of the implant 100 within the left atrial appendage by increasing anchoring member engagement with the lateral wall of the left atrial appendage.

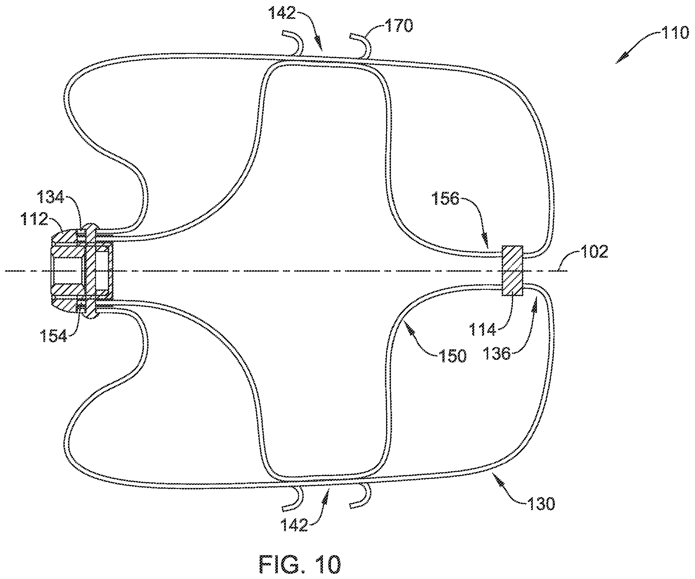

According to the invention, an outer surface of the second framework portion 150 abuts an inner surface of the first framework portion 130 in the expanded configuration, as seen in FIG. 10. In some embodiments, the outer surface of the second framework portion 150 may abut the inner surface of the first framework portion 130 proximate the midsection 142 of the first framework portion 130 in the expanded configuration. In some embodiments, the second framework portion 150 may be configured to support the first framework portion 130 proximate and/or adjacent to the midsection 142 of the first framework portion 130. In some embodiments, the second framework portion 150 may be configured to increase a radially outward force exerted on the lateral wall of the left atrial appendage by the first framework portion 130 proximate and/or adjacent to the midsection 142 of the first framework portion 130 to improve shaping and/or conformation of the lateral wall of the left atrial appendage to the outer shape of the first framework portion 130 and/or to improve anchoring member engagement with the lateral wall of the left atrial appendage. In some embodiments, the first framework portion 130 may include at least one anchoring member 170 extending radially outward from the first framework portion 130 proximate the midsection 142 of the first framework portion 130. In some embodiments, the at least one anchoring member 170 may be configured to engage with the lateral wall of the main body of the left atrial appendage. In some embodiments, the at least one anchoring member 170 may be formed as J-shaped hooks having a free end extending in and/or directed toward a proximal direction with respect to the longitudinal axis 102 of the expandable framework 110. Other configurations are also contemplated. In some embodiments, one of, some of, and/or all of the at least one anchoring member 170 may extend through the occlusive element 120, where present, as shown in FIG. 4. According to the invention, at least one anchoring member 170 is aligned longitudinally with the outer surface of the second framework portion 150 abutting the inner surface of the first framework portion 130, as seen in FIG. 10. In some embodiments, the second framework portion 150 may be configured to improve fixation of the implant 100 within the left atrial appendage by increasing anchoring member engagement with the lateral wall of the left atrial appendage.

A method for occluding the left atrial appendage may comprise advancing the implant 100 into the left atrial appendage. For example, the implant 100 may be advanced to the left atrial appendage within the lumen 42 of the delivery sheath 40 in the collapsed configuration. The method includes deploying the expandable framework 110 from the delivery sheath 40 within the left atrial appendage. The method further includes expanding and/or shifting the expandable framework 110 from the collapsed configuration to the expanded configuration within the left atrial appendage. In the expanded configuration, the expandable framework 110 may be urged into contact with, engaged with, and/or anchored to the lateral wall of the main body of the left atrial appendage. In at least some embodiments, the implant 100 and/or the expandable framework 110 may span across the ostium of the left atrial appendage. In some embodiments, the implant 100 and/or the expandable framework 110 may span completely across the ostium of the left atrial appendage, thereby effectively removing the left atrial appendage from the circulatory system of the patient.

When satisfied with the positioning of the implant 100 within the left atrial appendage, the core wire 30 may be disconnected from the implant 100, thereby leaving the implant 100 disposed at and/or in the left atrial appendage. In some embodiments, disconnecting the core wire 30 from the implant 100 may include rotating the externally threaded distal end of the core wire 30 relative to the implant 100 and/or the proximal hub 112 to disengage the core wire 30 from the implant 100.

In some embodiments, the delivery sheath 40 and/or the core wire 30 may include a keying structure configured to prevent rotation of the core wire 30 relative to the proximal hub 112. In such embodiments, the keying structure is disengaged prior to rotating the core wire 30 relative to the implant 100 and/or the proximal hub 112. When the keying structure is engaged, rotation of the core wire 30 may be transmitted to implant 100 and/or the expandable framework 110. In some embodiments, rotation of the implant 100 and/or the expandable framework 110 may facilitate positioning and/or orientation of the implant 100 and/or the expandable framework 110 relative to the left atrial appendage, for example, with respect to an asymmetrical and/or irregular ostium and/or left atrial appendage. Other configurations, purposes, and/or results are also contemplated.

The materials that can be used for the various components of the system (and/or other elements disclosed herein) and the various components thereof disclosed herein may include those commonly associated with medical devices and/or systems. For simplicity purposes, the following discussion makes reference to the system. However, this is not intended to limit the devices and methods described herein, as the discussion may be applied to other elements, members, components, or devices disclosed herein, such as, but not limited to, the implant, the delivery sheath, the core wire, the expandable framework, the occlusive element, etc. and/or elements or components thereof.

**In** some embodiments, the system and/or components thereof may be made from a metal, metal alloy, polymer (some examples of which are disclosed below), a metal-polymer composite, ceramics, combinations thereof, and the like, or other suitable material.

Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the system and/or other elements disclosed herein may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the system and/or other elements disclosed herein. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the system and/or other elements disclosed herein to achieve the same result.

In some embodiments, a degree of Magnetic Resonance Imaging (MRI) compatibility is imparted into the system and/or other elements disclosed herein. For example, the system and/or components or portions thereof may be made of a material that does not substantially distort the image and create substantial artifacts (e.g., gaps in the image). Certain ferromagnetic materials, for example, may not be suitable because they may create artifacts in an MRI image. The system or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the system and/or other elements disclosed herein may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), MARLEX^{®} high-density polyethylene, MARLEX^{®} low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the system and/or other elements disclosed herein may include a fabric material disposed over or within the structure. The fabric material may be composed of a biocompatible material, such a polymeric material or biomaterial, adapted to promote tissue ingrowth. In some embodiments, the fabric material may include a bioabsorbable material. Some examples of suitable fabric materials include, but are not limited to, polyethylene glycol (PEG), nylon, polytetrafluoroethylene (PTFE, ePTFE), a polyolefinic material such as a polyethylene, a polypropylene, polyester, polyurethane, and/or blends or combinations thereof.

In some embodiments, the system and/or other elements disclosed herein may include and/or be formed from a textile material. Some examples of suitable textile materials may include synthetic yarns that may be flat, shaped, twisted, textured, preshrunk or un-shrunk. Synthetic biocompatible yarns suitable for use in the present disclosure include, but are not limited to, polyesters, including polyethylene terephthalate (PET) polyesters, polypropylenes, polyethylenes, polyurethanes, polyolefins, polyvinyls, polymethylacetates, polyamides, naphthalene dicarboxylene derivatives, natural silk, and polytetrafluoroethylenes. Moreover, at least one of the synthetic yarns may be a metallic yarn or a glass or ceramic yarn or fiber. Useful metallic yarns include those yarns made from or containing stainless steel, platinum, gold, titanium, tantalum or a Ni-Co-Cr-based alloy. The yarns may further include carbon, glass or ceramic fibers. Desirably, the yarns are made from thermoplastic materials including, but not limited to, polyesters, polypropylenes, polyethylenes, polyurethanes, polynaphthalenes, polytetrafluoroethylenes, and the like. The yarns may be of the multifilament, monofilament, or spun types. The type and denier of the yarn chosen may be selected in a manner which forms a biocompatible and implantable prosthesis and, more particularly, a vascular structure having desirable properties.

In some embodiments, the system and/or other elements disclosed herein may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl ketone, an RGD peptidecontaining compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vasoactive mechanisms.

It should be understood that this disclosure is, in many respects, only illustrative. Changes may be made in details, particularly in matters of shape, size, and arrangement of steps, without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. An implant (100) for occluding a left atrial appendage, comprising:
an expandable framework (110) including a first framework portion (130) and a second framework portion (150), wherein the expandable framework (110) is configured to shift along a longitudinal axis between a collapsed configuration and an expanded configuration;
wherein the second framework portion (150) is disposed radially inward of the first framework portion (130) in the collapsed configuration and an outer surface of the second framework portion (150) abuts an inner surface of the first framework portion (130) in the expanded configuration;
wherein the expandable framework includes a proximal hub (112) and a distal hub (114);
wherein the first framework portion is fixedly attached to the proximal hub (112) and the distal hub (114);
wherein the second framework portion is fixedly attached to the proximal hub (112) and the distal hub (114),
**characterized in that** the first framework portion (130) includes at least one anchoring member (170) extending radially outward from the first framework portion (130), wherein the at least one anchoring member (170) is aligned longitudinally with the outer surface of the second framework portion (150) abutting the inner surface of the first framework portion (130).

2. The implant (100) of claim 1, wherein the first framework portion (130) includes a first plurality of interconnected struts (132) formed from a first tubular member;
wherein the second framework portion (150) includes a second plurality of interconnected struts (152) formed from a second tubular member.

3. The implant (100) of any one of claims 1-2, wherein a portion of the second framework portion (150) is disposed radially outward of the first framework portion (130) in the expanded configuration.

4. The implant (100) of claim 3, wherein the portion of the second framework portion (150) disposed radially outward of the first framework portion (130) includes at least one anchoring member (170) extending radially outward from the second framework portion (150).

5. The implant (100) of claim 3, wherein the portion of the second framework portion (150) disposed radially outward of the first framework portion (130) is disposed adjacent a proximal shoulder (140) of the first framework portion (130).

6. The implant (100) of claim 3, wherein the portion of the second framework portion (150) disposed radially outward of the first framework portion (130) is disposed proximate a midsection of the first framework portion (130).

7. The implant (100) of any one of claims 1 to 6, wherein the outer surface of the second framework portion (150) abuts the inner surface of the first framework portion (150) adjacent a proximal shoulder (140) of the first framework portion (130).

8. The implant (100) of any one of claims 1 to 6, wherein the outer surface of the second framework portion (180) abuts the inner surface of the first framework portion (130) proximate a midsection of the first framework portion (130).

9. The implant (100) of any one of claims 1-8, wherein the second framework portion (150) is more compliant than the first framework portion (130).

10. The implant (100) of any one of claims 1-9, further comprising an occlusive element (120) disposed on the expandable framework (110).

11. A system (10) for occluding a left atrial appendage, comprising:
a delivery sheath (40) having a lumen (42);
the implant (100) of any one of claims 1-10; and
a core wire (30) releasably securable to the proximal hub of the implant (100).

## Patentansprüche

1. Implantat (100) zum Verschließen eines linken Herzohrs, aufweisend:
ein expandierbares Rahmenwerk (110) mit einem ersten Rahmenwerkabschnitt (130) und einem zweiten Rahmenwerkabschnitt (150), wobei das expandierbare Rahmenwerk (110) konfiguriert ist, entlang einer Längsachse zwischen einer kollabierten Konfiguration und einer expandierten Konfiguration zu wechseln;
wobei in der kollabierten Konfiguration der zweite Rahmenwerkabschnitt (150) radial innerhalb des ersten Rahmenwerkabschnitts (130) angeordnet ist und in der expandierten Konfiguration eine Außenoberfläche des zweiten Rahmenwerkabschnitts (150) an einer Innenoberfläche des ersten Rahmenwerkabschnitts (130) anliegt;
wobei das expandierbare Rahmenwerk eine proximale Nabe (112) und eine distale Nabe (114) aufweist;
wobei der erste Rahmenwerkabschnitt fest an der proximalen Nabe (112) und der distalen Nabe (114) angebracht ist;
wobei der zweite Rahmenwerkabschnitt fest an der proximalen Nabe (112) und der distalen Nabe (114) angebracht ist,
**dadurch gekennzeichnet, dass** der erste Rahmenwerkabschnitt (130) mindestens ein Verankerungselement (170) aufweist, das sich von dem ersten Rahmenwerkabschnitt (130) radial nach außen erstreckt, wobei das mindestens eine Verankerungselement (170) longitudinal mit der an der Innenoberfläche des ersten Rahmenwerkabschnitts (130) anliegenden Außenoberfläche des zweiten Rahmenwerkabschnitts (150) ausgerichtet ist.

2. Implantat (100) nach Anspruch 1, wobei der erste Rahmenwerkabschnitt (130) mehrere erste miteinander verbundene Streben (132) aufweist, die aus einem ersten röhrenförmigen Element gebildet sind;
wobei der zweite Rahmenwerkabschnitt (150) mehrere zweite miteinander verbundene Streben (152) aufweist, die aus einem zweiten röhrenförmigen Element gebildet sind.

3. Implantat (100) nach einem der Ansprüche 1 bis 2, wobei in der expandierten Konfiguration ein Teil des zweiten Rahmenwerkabschnitts (150) radial außerhalb des ersten Rahmenwerkabschnitts (130) angeordnet ist.

4. Implantat (100) nach Anspruch 3, wobei der radial außerhalb des ersten Rahmenwerkabschnitts (130) angeordnete Teil des zweiten Rahmenwerkabschnitts (150) mindestens ein Verankerungselement (170) aufweist, das sich von dem zweiten Rahmenwerkabschnitt (150) radial nach außen erstreckt.

5. Implantat (100) nach Anspruch 3, wobei der radial außerhalb des ersten Rahmenwerkabschnitts (130) angeordnete Teil des zweiten Rahmenwerkabschnitts (150) angrenzend an eine proximale Schulter (140) des ersten Rahmenwerkabschnitts (130) angeordnet ist.

6. Implantat (100) nach Anspruch 3, wobei der radial außerhalb des ersten Rahmenwerkabschnitts (130) angeordnete Teil des zweiten Rahmenwerkabschnitts (150) nahe an einem Mittelabschnitt des ersten Rahmenwerkabschnitts (130) angeordnet ist.

7. Implantat (100) nach einem der Ansprüche 1 bis 6, wobei die Außenoberfläche des zweiten Rahmenwerkabschnitts (150) an der Innenoberfläche des ersten Rahmenwerkabschnitts (150) angrenzend an eine proximale Schulter (140) des ersten Rahmenwerkabschnitts (130) anliegt.

8. Implantat (100) nach einem der Ansprüche 1 bis 6, wobei die Außenoberfläche des zweiten Rahmenwerkabschnitts (180) an der Innenoberfläche des ersten Rahmenwerkabschnitts (130) nahe an einem Mittelabschnitt des ersten Rahmenwerkabschnitts (130) anliegt.

9. Implantat (100) nach einem der Ansprüche 1 bis 8, wobei der zweite Rahmenwerkabschnitt (150) flexibler als der erste Rahmenwerkabschnitt (130) ist.

10. Implantat (100) nach einem der Ansprüche 1 bis 9, ferner aufweisend ein an dem expandierbaren Rahmenwerk (110) angeordnetes Verschlusselement (120).

11. System (10) zum Verschließen eines linken Herzohrs, aufweisend:
einen Zuführungsschaft (40) mit einem Lumen (42);
das Implantat (100) nach einem der Ansprüche 1 bis 10; und
einen Kerndraht (30), der an der proximalen Nabe des Implantats (100) lösbar befestigbar ist.

## Revendications

1. Implant (100) pour boucher un appendice auriculaire gauche comprenant :
un cadre expansible (110) comprenant une première partie de cadre (130) et une deuxième partie de cadre (150), dans lequel le cadre expansible (110) est configuré pour se déplacer le long d'un axe longitudinal entre une configuration repliée et une configuration expansée ;
dans lequel la deuxième partie de cadre (150) est disposée radialement vers l'intérieur de la première partie de cadre (130) dans la configuration repliée et une surface externe de la deuxième partie de cadre (150) vient en butée contre une surface interne de la première partie de cadre (130) dans la configuration expansée ;
dans lequel le cadre expansible comprend un moyeu proximal (112) et un moyeu distal (114) ;
dans lequel la première partie de cadre est fixement fixée au moyeu proximal (112) et au moyeu distal (114) ;
dans lequel la deuxième partie de cadre est fixement fixée au moyeu proximal (112) et au moyeu distal (114),
**caractérisé en ce que** la première partie de cadre (130) comprend au moins un élément d'ancrage (170) s'étendant radialement vers l'extérieur à partir de la première partie de cadre (130), dans lequel le au moins un élément d'ancrage (170) est aligné, de manière longitudinale, avec la surface externe de la deuxième partie de cadre (150) qui vient en butée contre la surface interne de la première partie de cadre (130).

2. Implant (100) selon la revendication 1, dans lequel la première partie de cadre (130) comprend une première pluralité d'entretoises interconnectées (132) formées à partir d'un premier élément tubulaire ;
dans lequel la deuxième partie de cadre (150) comprend une deuxième pluralité d'entretoises interconnectées (152) formées à partir d'un deuxième élément tubulaire.

3. Implant (100) selon l'une quelconque des revendications 1 à 2, dans lequel une partie de la deuxième partie de cadre (150) est disposée radialement vers l'extérieur de la première partie de cadre (130) dans la configuration expansée.

4. Implant (100) selon la revendication 3, dans lequel la partie de la deuxième partie de cadre (150) disposée radialement vers l'extérieur de la première partie de cadre (130) comprend au moins un élément d'ancrage (170) s'étendant radialement vers l'extérieur à partir de la deuxième partie de cadre (150).

5. Implant (100) selon la revendication 3, dans lequel la partie de la deuxième partie de cadre (150) disposée radialement vers l'extérieur de la première partie de cadre (130) est disposée de manière adjacente à un épaulement proximal (140) de la première partie de cadre (130).

6. Implant (100) selon la revendication 3, dans lequel la partie de la deuxième partie de cadre (150) disposée radialement vers l'extérieur de la première partie de cadre (130) est disposée à proximité d'une section médiane de la première partie de cadre (130).

7. Implant (100) selon l'une quelconque des revendications 1 à 6, dans lequel la surface externe de la deuxième partie de cadre (150) vient en butée contre la surface interne de la première partie de cadre (150) adjacente à un épaulement proximal (140) de la première partie de cadre (130).

8. Implant (100) selon l'une quelconque des revendications 1 à 6, dans lequel la surface externe de la deuxième partie de cadre (180) vient en butée contre la surface interne de la première partie de cadre (130) à proximité d'une section médiane de la première partie de cadre (130).

9. Implant (100) selon l'une quelconque des revendications 1 à 8, dans lequel la deuxième partie de cadre (150) est plus souple que la première partie de cadre (130).

10. Implant (100) selon l'une quelconque des revendications 1 à 9, comprenant en outre un élément occlusif (120) disposé sur le cadre expansible (110).

11. Système (10) pour boucher un appendice auriculaire gauche, comprenant :
une gaine de pose (40) ayant une lumière (42) ;
l'implant (100) selon l'une quelconque des revendications 1 à 10 ; et
un fil central (30) pouvant être fixé de manière amovible au moyeu proximal de l'implant (100).
